# EUROPEAN PATENT SPECIFICATION

(11) **EP 4 321 843 B1**
(45) Date of publication and mention of the grant of the patent: **25.02.2026**
(21) Application number: 21931980.3
(22) Date of filing: 26.03.2021
(51) Int. Cl.: G01F 1/66, G01F 15/14, G01F 23/28, G01P 5/24, F16B 41/00, G01D 11/24, G01D 11/30, G01F 1/002, G01N 33/18, G01F 15/061, G01F 23/284, G01F 23/2962, G01F 15/00, G01F 15/06

(54) **VANDAL-PROOF INSTALLATION SYSTEM FOR THE MONITORING OF PHYSICAL VARIABLES IN WATER, COMPRISING: A FIRST MEMBER; A SECOND MEMBER; A THIRD MEMBER AND A FOURTH MEMBER; WHERE THE FIRST MEMBER COMPRISES A PLURALITY OF COMPARTMENTS FOR HOUSING A PLURALITY OF DEVICES. ASSEMBLY METHOD**
VANDALSICHERES INSTALLATIONSSYSTEM MIT EINEM ERSTEN ELEMENT, EINEM DRITTEN ELEMENT UND EINEM VIERTEN ELEMENT. MONTAGEVERFAHREN
SYSTÈME DE MONTAGE ANTI-VANDALISME POUR LA SURVEILLANCE DE VARIABLES PHYSIQUES DE L'EAU QUI COMPREND UN PREMIER ÉLÉMENT, UN DEUXIÈME ÉLÉMENT, UN TROISIÈME ÉLÉMENT ET UN QUATRIÈME ÉLÉMENT; LE PREMIER ÉLÉMENT COMPRENANT UNE PLURALITÉ DE COMPARTIMENTS POUR LOGER UNE PLURALITÉ DE DISPOSITIFS, PROCÉDÉ DE MONTAGE

(43) Date of publication of application: 14.02.2024
(73) Proprietor: Capta Hydro SpA, Santiago (CL)
(72) Inventor: DE LA JARA HARTWIG, Emilio Alfonso, 5930 La Florida Santiago (CL); ECHEVERRÍA LAVÍN, Rodrigo, Las Condes Santiago (CL)
(74) Representative: Garcia González, Sergio
(86) International application number: PCT/CL2021/050021
(87) International publication number: WO 2022/198345

(56) References cited:
- WO-A1-2019/155222
- WO-A1-2020/113275
- WO-A1-2021/024174
- CN-A- 108 871 423
- CN-U- 206 758 134
- CN-U- 209 043 396
- CN-U- 209 117 097
- CN-U- 209 878 042
- CN-U- 210 221 150
- CN-U- 210 321 881
- CN-U- 212 693 013
- US-A- 5 633 809
- CAPTAHYDRO, 2019, XP055973835, Retrieved from the Internet <URL:https://www.thinkagro.cl/wp-content/uploads/2021/03/8-CaptaHydro-1.pdf> [retrieved on 20211104]

## Description

The present invention relates to the field of channel monitoring systems and, specifically, to an anti-vandalism mounting system for monitoring water physical variables in natural and artificial open channels.

The assembly of the components of the described anti-vandalism system allows, among other things, to protect the inner elements that allow the monitoring of the water from theft and damage that they may suffer from third parties or from the severe weather present in the installation location, as well as allowing the improvement of the accuracy in the system telemetry thanks to the thermal insulation provided, therefore, not only providing a safer system, but also providing a system that offers a better performance in operation compared to the currently available solutions.

The system of the invention essentially comprises a first member, comprising a base with a plurality of perforations to introduce a plurality of anchoring means to fix the first member to an installation surface of the system; a second member, which is fixed on the first member of the system by means of a plurality of anchoring means; a third member arranged externally, which is attached to the first and second members from inside the system by means of anchoring means; and a fourth member pivotally arranged at the bottom of the third member; wherein the first member comprises a plurality of compartments to house a plurality of devices for the operation of the system and for the monitoring of physical variables to be protected by the system; and wherein the system comprises an energy generating device and a plurality of safety devices, so that the fourth member is fixed to the third member of the system.

Preferably, the devices for operating the system and for monitoring physical variables correspond to a plurality of batteries, at least one anti-humidity device, at least one energy, measurement and telecommunications controller device, at least one ultrasonic sensor, and at least one wireless communication antenna; wherein the energy generating device comprises the fourth member, which also comprises an impact resistant cover on said energy generator, wherein both the energy generating device and the impact resistant cover are supported by a rear support of the energy generating device.

In addition, the invention comprises a procedure for assembling the anti-vandalism mounting system for monitoring the water physical variables in open channels, as described above.

### BACKGROUND

The technology for remote monitoring or telemetry, specifically to measure physical variables in surface water distribution networks in natural and artificial open channels, has made great advances in recent decades, but it still has several challenges to overcome in order to be a cost-effective and robust solution. The physical variables that are of most interest for monitoring the distribution of water in open channels are flow and runoff level, in addition to other variables related to water chemistry. In this sense, the main problems for the measurement of this type of variables are the precision in time and the vandalism of its components, to which the components of these systems are exposed.

Currently, one of the main reasons that causes a low number of telemetry points in a large part of the networks of natural and/or artificial channels, as mentioned above, is the destruction or theft these facilities suffer by third parties, who see an opportunity to illicitly obtain the measurement equipment and other elements found within these systems, to commercialise them subsequently. This is largely due to the solitude in which these systems are arranged, along channels where there is usually no transit of people. This, added to the darkness of these places at night, since they do not have lighting, allows these individuals to infringe the weak security offered by the measurement systems to appropriate their components. Additionally, the use of one or more solar panels on a mast to supply energy to the electrical components of the system, notably increases the visibility of this equipment, increasing the probability of theft, as well as the cleaning that must be carried out on said panels so that they do not lose efficiency.

Another factor that affects the deterioration of these facilities is the environmental characteristics of the places where they are located, which can cause in a short period of time the malfunction of one or more of the components of the system due to pollution, high/low temperatures and/or humidity present in the environment.

As a consequence of these problems, frequent reviews and maintenance are necessary, in order to check the operating status of the system components and to verify that none of them have been infringed or damaged by third parties.

In this context, the solutions currently used for flow measurement in open channels are varied, but only a few are used for remote monitoring (telemetry). These solutions correspond to the methods of: volumetric capacity, gravimetric, chemical tracers, Gauckler-Manning equation, velocity area and measurement by hydraulic structure (gutters and landfills), the last two methods being the most used for flow telemetry.

The volumetric and gravimetric capacity is generally used in punctual measurements, manually, taking an instantaneous measurement of the flow (point measurement) and not permanently. The chemical tracer measurement is also used for instantaneous measurements and not permanently, with the disadvantage of requiring the provision of a chemical mass to measure, in addition to the periodic re-calibration of the measurement instruments. On the other hand, flow measurement by Gauckler-Manning equations is rarely used for flow monitoring, since its coefficient (Gauckel-Manning coefficient) varies over time, which causes the flow measurement of runoff height measurement, become less and less accurate. Regarding the area-velocity method for the measurement of flows in open flow, the most used types of sensors are the doppler and the one of transit time with multiple transducers, which must be fixed in some way in the bottom or in one of the walls on the side of the open channel, having problems regarding their safety as they are totally exposed. Finally, the most widely used method today is the measurement of flow from the measurement by hydraulic structure, due to its robustness and simplicity of the method. In this type of measurement, there is a unique relationship between height and flow (discharge curve) thanks to the transition from sub-critical to supercritical flow, allowing the isolation of downstream hydraulic conditions. In this way, the flow can be monitored continuously, only from the critical runoff height measurement, which is generally done thanks to an ultrasonic sensor or pressure sensor, which can be through a stilling or directly on the free surface of the water in the channel.

However, the hydraulic structure measurement method also has several unsolved problems. First, to carry out the construction of the stilling wells, it is necessary to stop or bypass the flow of the channel to build the adduction pipes. The construction of this well affects the cost and time of the construction of this work, in addition to the indirect costs due to the stoppage of the channel. A booth can be built on the stilling well or a padlocked drum can be installed to contain and protect the elements that measure and record the level of the well, requiring in both cases that the power supply system (photovoltaic panel) and antenna for communication is installed separately, as described in the Chinese Utility Model CN202092719U, which discloses a water level telemetry device using a level sensor powered by a photovoltaic solar generation system. One of the problems of having the photovoltaic panel is the remote visibility that is given to the measurement equipment due to its height installation, which could draw the attention of people who are interested in stealing one or more of the components or in vandalizing the facilities. In addition, these systems are exposed to possible damage of the solar panel through hail or birds strike damage.

Additionally, in the case of stilling wells with a steel cover, a stratification of the temperature of the air inside is generated inside the steel drum, causing that the temperature compensation of the transit time ultrasonic sensor is not representative of the average temperature of the air between water and sensor, decreasing its accuracy during the day and generating erroneous measurements. Additionally, a singularity of the stilling wells is the characteristic of having a rate close to zero inside, which affects the accumulation of solid sediments and proliferation of algae, leaving the well unable to function correctly due to the blockage of the adduction pipes of the well. One of the ways to mitigate this effect is by positioning the adduction pipe at a higher height making it impossible to measure the level of runoff for low volumes of flow which are below the midline of this pipe.

However, regarding the measurement of the level of runoff by hydraulic structure measured directly on the channel, there are several developments that have addressed this concept due to its simplicity in relatively narrow channels. One of the difficulties of this concept is the requirement to have supports on both sides of the channel which is difficult in relatively wide channels (for example, channels of more than 6 meters) requiring an assembly structure that requires a truck for its transport and perhaps a crane for assembly. Examples of this type of installation can be seen in US Patents US6907779B1 and US8474327B2. Patent US6907779B1 describes a continuous flow measurement recorder for measuring water flow in an open channel wherein an ultrasonic sensor obtains upstream measurements in an artificial channel arranged in the open channel. On the other hand, Patent US8474327B2 teaches an acoustic flow measuring set for pipes or open channels, through an acoustic transducer to measure the fluid rate wherein it is observed that in both Patents the components of the respective systems may be easily vandalized or damaged due to the action of third parties or environmental conditions.

In addition, none of the state-of-the-art systems whether those that use stilling wells or the like manages to provide a satisfactory solution to the problem of the distance measurement error between the sensor and the free surface of the water. This problem is caused by differences in air temperature in the path of the ultrasonic wave, so by taking a wrong reference temperature, the speed of sound used to calculate the distance will be wrong. This is mainly because the characteristics of the locations where the sensors are installed do not allow sufficient thermal insulation or do not have a component arrangement allowing them to measure a reference temperature closer to the average temperature between the sensor and the water.

Finally, in relation to flow telemetry based on the area-velocity method this can be done in many ways. The most used methods are transit time, consistent Doppler, inconsistent Doppler, laser Doppler, among others. A common element of all these forms of measurement is that the assembly of its measurement components must be carried out on the walls or at the bottom of the channel separately from the place where the interpretation of the signals, recording and sending of data is carried out. This way of arranging the components in the channel and on its side leaves them exposed to theft often requiring the fencing of the measurement area to prevent theft or damage of this equipment.

Therefore, it is necessary to have an anti-vandalism mounting system for monitoring the water physical variables in open channels offering a comprehensive solution in terms of accurate and reliable measurement of the water physical variables, as well as a compact and safe design which allows the user to trust in the autonomy and robustness of the system, allowing the installation of multiple systems along a channel without the concern of undertaking constant inspections and/or repairs to its components thus avoiding, among other things, the need to stop the channel to add a measurement or the intervention of the same with additional civil works required for the operation of the system thus providing a system that can be quickly installed in the operation site without the need for specific equipment or qualified personnel.

WO2020/113275 provides a system with a housing for a sensor array comprising: a mounting plate for mounting the housing to a surface; a cover attachable to the mounting plate, where the mounting plate and the cover are shaped to form an internal cavity within the housing; a sensor array contained within the internal cavity; and at least one air pathway in connection with the sensor array to enable air to pass from outside the housing to the sensor array.

### DESCRIPTION OF THE INVENTION

The present invention relates to an anti-vandalism mounting system for monitoring the water physical variables in natural and artificial open channels which allows measurements to be carried out in an environment free of physical and climatic interference, in addition to provide safety and autonomy to its components avoiding damage or theft of one or more of them.

In this sense, according to a preferred embodiment of the invention, the anti-vandalism mounting system for monitoring the water physical variables in open channels comprises:
- a first member, comprising a base with a plurality of perforations for introducing a plurality of anchoring means for fixing the first member to an installation surface of the system;
- a second member, which is fixed on the first member of the system, by means of a plurality of anchoring means;
- a third member, arranged externally which is attached to the first and second members from inside the system by anchoring means; and
- a fourth member, pivotally arranged in the lower part of the third member;

wherein the first member comprises a plurality of compartments to house a plurality of devices for the operation of the system and for the monitoring of physical variables to be protected by the system; and
wherein the system comprises an energy generating device and a plurality of safety devices, so that the fourth member is fixed to the third member of the system.

This embodiment of the invention makes it possible to obtain a compact and low-visibility system, which through the assembly of its four members provides a solution that makes it much more difficult for third parties to infringe or steal one or more of its components.

According to another embodiment of the invention, the devices for operating the system and for monitoring physical variables correspond to a plurality of batteries, at least one anti-humidity device, at least one energy, measurement and telecommunications controller device, at least one ultrasonic sensor, and at least one wireless communication antenna.

According to another embodiment of the invention, the energy generating device is a photovoltaic solar panel.

According to another embodiment of the invention, the energy generating device comprises a fourth member, which also comprises an impact resistant cover on said energy generator, wherein both the energy generating device and the impact resistant cover are supported by a rear support of the energy generating device.

The fact that the energy generating device is a photovoltaic solar panel, and that it is inserted together with the system of the invention allows a complete anti-vandalism mounting system to be obtained, since all the components of the system are in a single unit protected by its different members and by the impact resistant cover which allows the photovoltaic solar panel to receive enough solar radiation to power the system batteries, not being necessary for said solar panel to be exposed, as in some solutions of the state of the art, wherein the solar panel is located on a post or the like, being easily reachable by third parties.

According to another embodiment of the invention, the devices for the operation of the system and for the monitoring of physical variables also comprise at least one camera to monitor the status of the channel, to verify the presence of garbage or foreign objects operating also as a means for verifying the height of water. This helps the system operator to visually monitor the operation of the system and the flow of the channel, being able to visually certify in case the system provides data that may suggest some inconvenience in the channel, such as water theft, obstruction by presence of waste, etc.

According to another embodiment of the invention, the system further comprises at least one element for measuring the height of water mounted on the third member.

According to another embodiment of the invention, the element for measuring the height of water is a radar device.

According to another embodiment of the invention, the system further comprises a module for measuring the runoff velocity profile in the channel, laterally or at the bottom of the channel, comprising two parts fixed to the inner members of the system and that supports a device with a plurality of transducers for the measurement of average speed by Doppler effect, transit time or other similar method, the module extending to the wall of the channel or the bottom, which is fixed to said wall of the channel by a plurality of anchoring means.

According to another embodiment of the invention, the system further comprises a housing element above the channel fixed to the inner members of the system by anchoring means, wherein a radar or ultrasonic water height measurement sensor and a device for the measurement of the superficial speed and height of runoff in the cannel, are mounted at its end.

According to another embodiment of the invention, the system further comprises an element above the channel fixed to the inner members of the system by anchoring means, wherein a radar or ultrasonic water height measurement sensor and a device for measuring the surface speed and runoff height in the cannel, are mounted at its end.

According to another embodiment of the invention, the system further comprises an additional module mounted in the lower part of the system fixed to it through anchoring means, wherein a radar or ultrasonic water height measurement sensor and a device for the measurement of the superficial speed and height of runoff in the cannel, are arranged inside it.

According to another embodiment of the invention, the system further comprises an arm, preferably horizontal, mounted in the lower part of the system fixed at one of its ends to said lower part through anchoring means, wherein an additional module is mounted at its other end fixed to it through anchoring means, wherein a radar or ultrasonic water height measurement sensor and a device for measuring the surface velocity and height of water runoff in the cannel, are arranged inside it. This arrangement prevents interference from occurring between the radar or ultrasonic water height measurement sensor and the channel wall, when it is arranged in the housing element anchored to the channel wall, wherein the sound or electromagnetic wave can be interfered by its proximity to the wall, by hydraulic structures in the proximity to the wall or because the wall is an angled slope, when lowering the level of the cannel, the distance between the sensor and the water free surface would not be measured.

According to another embodiment of the invention, the installation surface of the system corresponds to the upper edge of one of the channel walls.

According to another embodiment of the invention, the installation surface of the system corresponds to a bridge that crosses the channel transversely and that is mounted on the upper edges of the channel walls through anchoring means.

The present invention also refers to a procedure for assembling an anti-vandalism mounting system for monitoring water physical variables in open channels based on the system described above, where the steps of the system components installation allow to obtain all the mentioned advantages, in terms of allowing an accurate and interference-free measurement, in addition to providing adequate security to avoid the destruction or theft of part or all the system.

According to this preferred embodiment of the invention, the assembly procedure of the anti-vandalism mounting system for monitoring the water physical variables in open channels, comprises the following stages:
(i) fixing a first member to an installation surface of the system by inserting a plurality of anchoring means into a plurality of perforations in said first member;
(ii) fixing a second member to the first member by means of a plurality of anchoring means;
(iii) fixing a third member, arranged externally, to the first and second members, from inside the system by anchoring means; and
(iv) pivotally arranging a fourth member in the lower part of the third member;
(v) housing a plurality of devices to be protected by the system in a plurality of compartments of the first member;
(vi) providing an energy generating device in the system; and
(vii) providing a plurality of safety devices in the fourth member, so that it is fixed to the third member of the system.

According to another embodiment of the invention, the step of arranging an energy generating device in the system further comprises arranging said energy generating device together with an impact resistant cover on said energy generating device in the fourth member, wherein the energy generating device corresponds to a photovoltaic solar panel.

According to another embodiment of the invention, the procedure further comprises arranging a rear support for the photovoltaic solar panel in the fourth member to support said photovoltaic solar panel and the impact resistant cover.

According to another embodiment of the invention, the procedure further comprises mounting in the third member at least one element for measuring the height of water.

According to another embodiment of the invention, the procedure further comprises assembling in the inner members of the system a module for measuring the runoff velocity profile in the channel, laterally or at the bottom of the channel, comprising two parts fixed to said inner members and which supports a device with a plurality of transducers for the measurement of speed by Doppler effect, transit time or other similar method, the module extending to the wall of the channel or the bottom, which is fixed to said channel wall by a plurality of anchoring means.

According to another embodiment of the invention, the procedure further comprises mounting an element above the channel on the inner members of the system, fixed to said inner members by anchoring means, wherein a radar or ultrasonic water height measurement and a device for measuring the surface velocity and runoff height in the cannel, are mounted at its end.

According to another embodiment of the invention, the procedure further comprises mounting an additional module to the lower part of the system fixed to it through anchoring means, wherein a radar or ultrasonic water height measurement sensor and a device for the measurement of the superficial speed and height of runoff in the cannel, are arranged inside it.

According to another embodiment of the invention, the procedure further comprises mounting an arm in the lower part of the system fixed at one of its ends to said lower part through anchoring means, wherein an additional module is mounted at its other end fixed to it through anchoring means, wherein a radar or ultrasonic water height measurement sensor and a device for measuring the surface speed and runoff height in the cannel, are arranged inside it.

From the above description, it is possible to appreciate that the invention provides several specific advantages, in addition to those already mentioned compared to the existing solutions:
- The problem of the low reliability of the stilling wells to measure the runoff height of the channel due to the accumulation of sediment and clogging in the well or the pipes that communicate the well with the cannel, is solved.
- The temperature stratification problem within the stilling well which causes non-representative measurements of the temperature for the calculation of the transit time, is solved.
- The construction of the stilling well to stabilize waves on the free surface of the channel is avoided, which means that the channel has to be stopped, in order to install, build a by-pass and modify its civil works, adding an additional cost to the construction of the work.
- The high visual impact of the solar panels and telemetry stations of the existing systems and facilities is avoided.
- Damage to the solar panel due to climatic effects (hail, branches colliding due to the action of the wind, among other effects) and the collision of birds against the surface of the panel, are avoided.
- The risk that the system or any component of it suffers acts of vandalism by third parties who wish to damage it or appropriate it, is reduced.

### BRIEF DESCRIPTION OF THE FIGURES

As part of the present invention, the following representative Figures are presented which show a preferred configuration of the invention and, therefore, should not be considered as limiting the definition of the claimed matter.
Figures 1 to 4 show a solution for channel monitoring of the state of the art;
Figures 5 to 7 show additional solutions for channel monitoring of the state of the art;
Figures 8 and 9 show an additional solution for channel monitoring of the state of the art;
Figures 10 and 11 show an isometric view of the main components decoupled from a first preferred configuration of the anti-vandalism mounting system for monitoring water physical variables arranged in an open channel;
Figures 12 and 13 show an isometric view of the first preferred configuration of the anti-vandalism mounting system for monitoring the physical variables of the water arranged in an open and assembled channel;
Figure 14 shows an isometric view of a second preferred configuration of the anti-vandalism mounting system for monitoring the water physical variables, arranged in an open channel according to a preferred configuration of the invention;
Figure 15 shows a view of the anchoring means of the system according to the first and second preferred configurations of the invention;
Figure 16 shows a view of the first member of the system, according to the second preferred configuration of the invention;
Figure 17 shows a view of the devices arranged in the first member of the system according to the second preferred configuration of the invention;
Figure 18 shows a view of the arrangement of the second member of the system according to the second preferred configuration of the invention;
Figure 19 shows a view of the arrangement of the third member of the system according to the second preferred configuration of the invention;
Figure 20 shows a view of the arrangement of the fourth member of the system according to the second preferred configuration of the invention;
Figure 21 shows a view of the arrangement of the photovoltaic solar panel of the system according to the second preferred configuration of the invention;
Figure 22 shows a view of the arrangement of the ultrasonic sensor and the camera of the system according to the second preferred configuration of the invention;
Figure 23 shows a view of the arrangement of the system displayed in Figure 21 mounted on a bridge over the channel with an additional anti-vandalism mounting module with thermal insulation which contains the measurement sensors of water height and surface velocity of the channel; and
Figure 24 shows a detailed view of the additional anti-vandalism mounting module with thermal insulation which contains the sensors for measuring the height of the water and the surface velocity of the channel, which can be seen in more detail in the bullet.

### DETAILED DESCRIPTION OF A PREFERRED EMBODIMENT

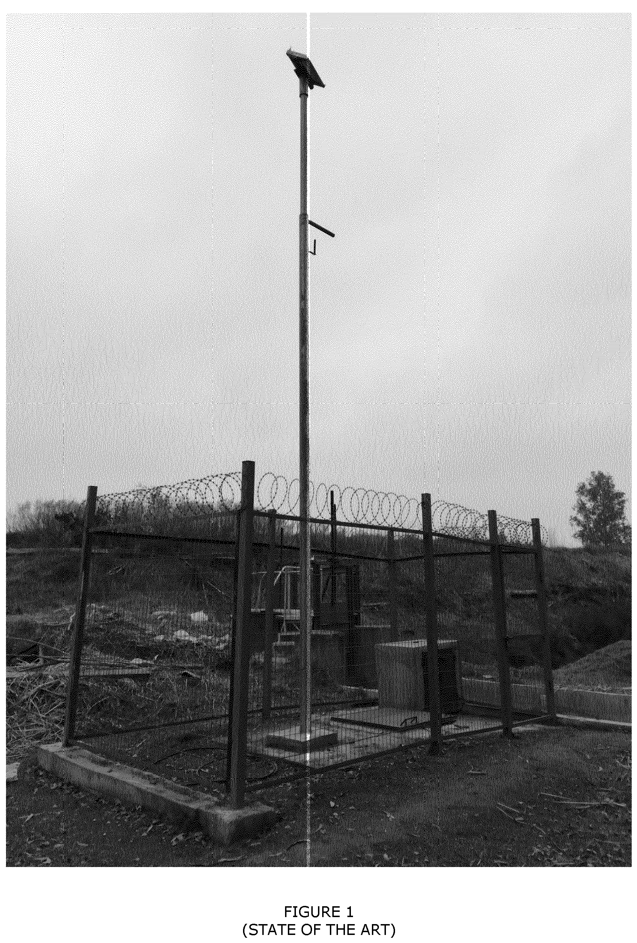

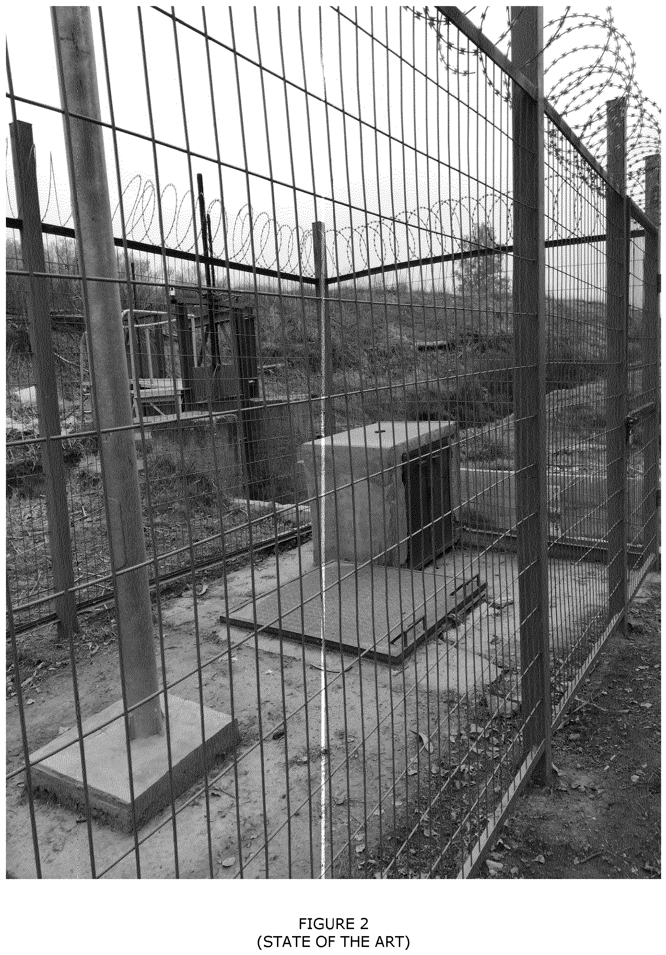

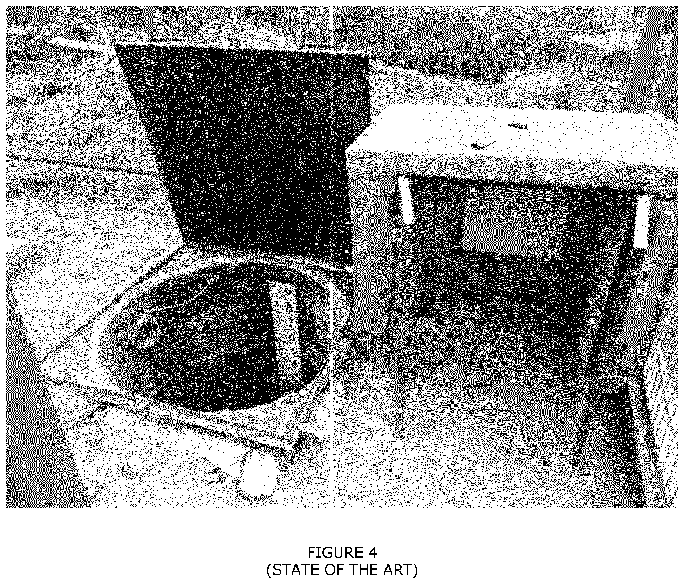

With reference to the accompanying Figures, Figures 1 to 4 show a solution for channel monitoring according to the state of the art, wherein a stilling well is used. In it, several problems can be observed which are solved from the present invention. One of them is related to the space covered by the installation of the system (Figures 1 and 2), which uses a large area that must be protected by a concrete civil work, added to other protection measures, such as bars, barbed wires, etc. In addition, the photovoltaic panel that powers the system is highly visible, increasing the chances of attracting third parties to the location of the system so that it can be vandalized.

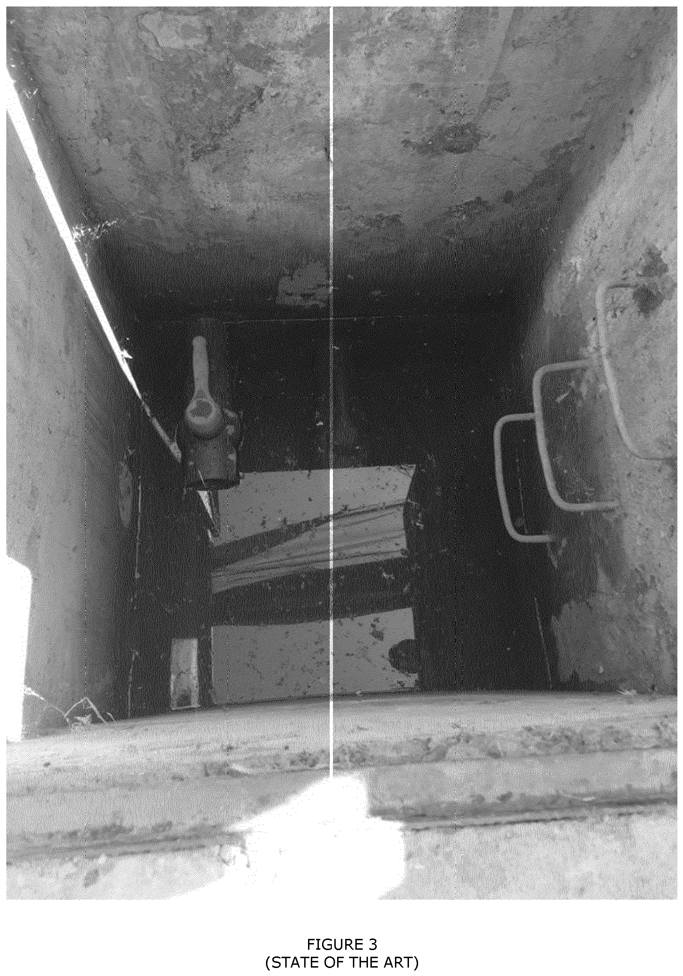

Figure 3 shows the stilling well of the system of Figure 1 which is dirty and full of sediment, this being another of the problems that these systems have not been able to address. The accumulated dirt causes the well adduction tube to become clogged preventing the true height of water of the channel from being reflected, in addition to not being able to determine the precise moment in which the transmission of the height of water from the channel to the stilling well was delayed or obstructed. In order to avoid this, this type of system must be continuously cleaned causing non insignificant expenses for this concept. In addition, it is important to note the level of civil works associated with the measurement, for which it is necessary to stop the channel to carry out said infrastructure, therefore, it is an expensive and time-consuming system to install.

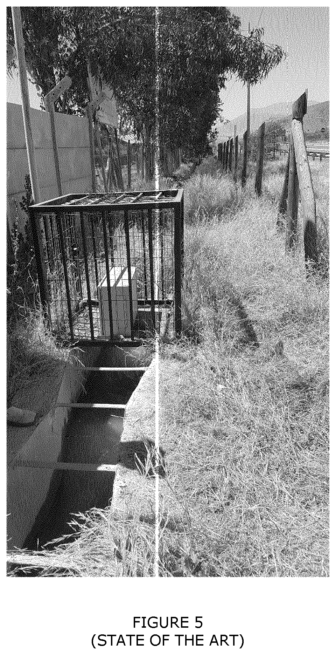

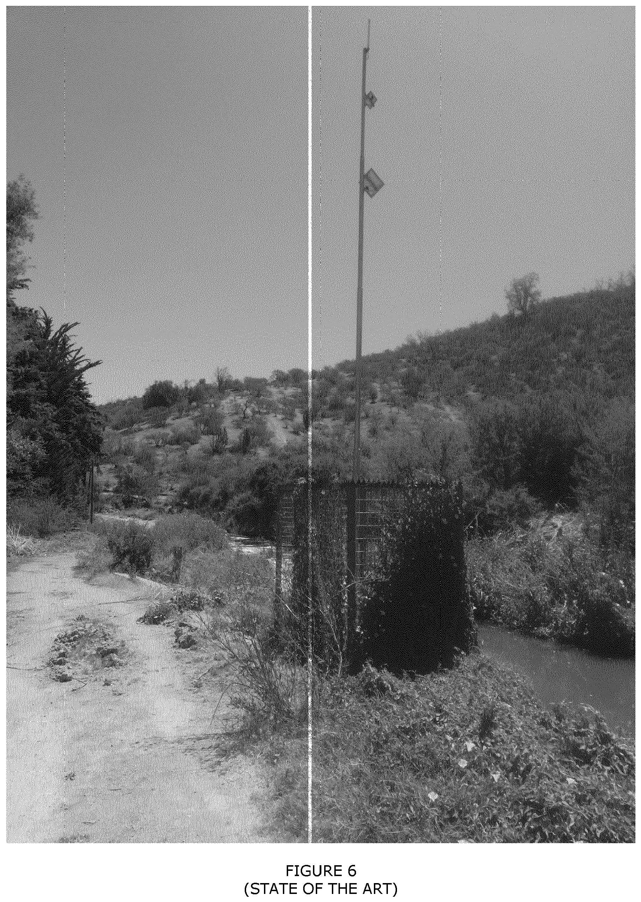

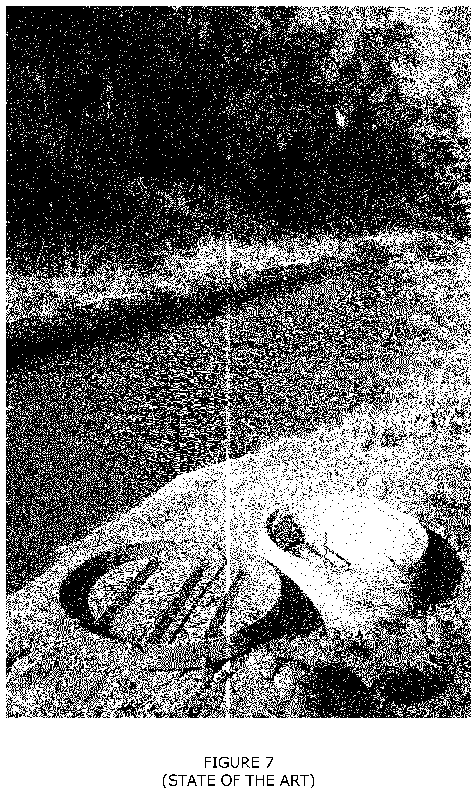

With respect to Figures 5 to 7, various solutions aiming to solve the problem of civil works associated with the monitoring system of the state of the art can be seen. However, it is possible to notice that, in any case, these solutions suffer from vandalism by third parties who manage to infringe the security systems of said systems to steal their components. This is mainly because these solutions, although they save space in their installation do not manage to take care of reducing the visibility of the solar panel which attracts third parties and/or they choose to reduce the level of protection of the system, being able to be easily infringed, for example, through a cutting tool or levering.

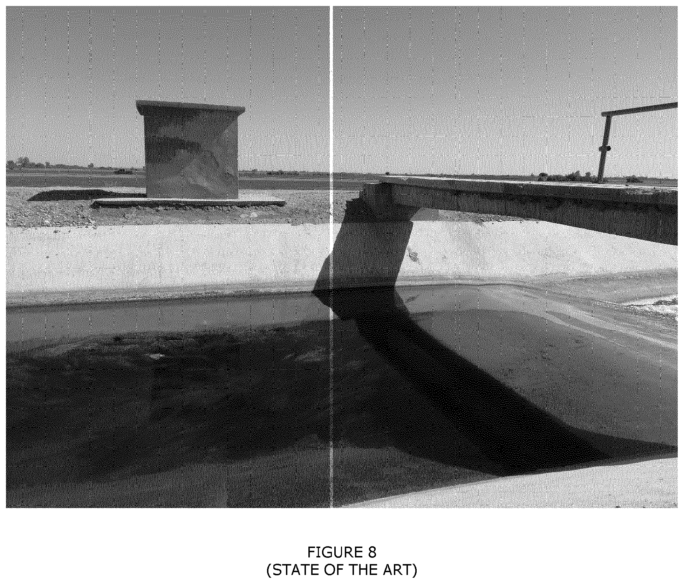

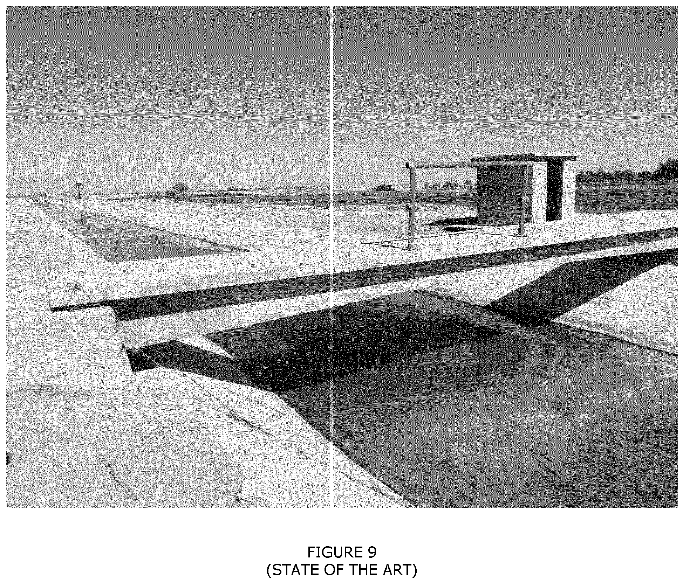

Finally, in Figures 8 and 9, a state-of-the-art monitoring station is observed with its adduction tubes completely covered due to the accumulation of algae showing the importance of constant maintenance in this type of system, so that can operate normally and accurately. In Figure 9 specifically, another view of the same monitoring station can be seen wherein it can be seen that the door was removed being completely vandalized from the inside. In addition, it is observed that in the same existing bridge in the place the railing was stolen practically in its entirety leaving only the central part, showing how strong vandalism is in these isolated points where it is not possible to carry out continuous and effective monitoring, and therefore, there is a need for safer and more reliable channel monitoring systems to maintain continuous and accurate monitoring of the channel without increasing installation costs.

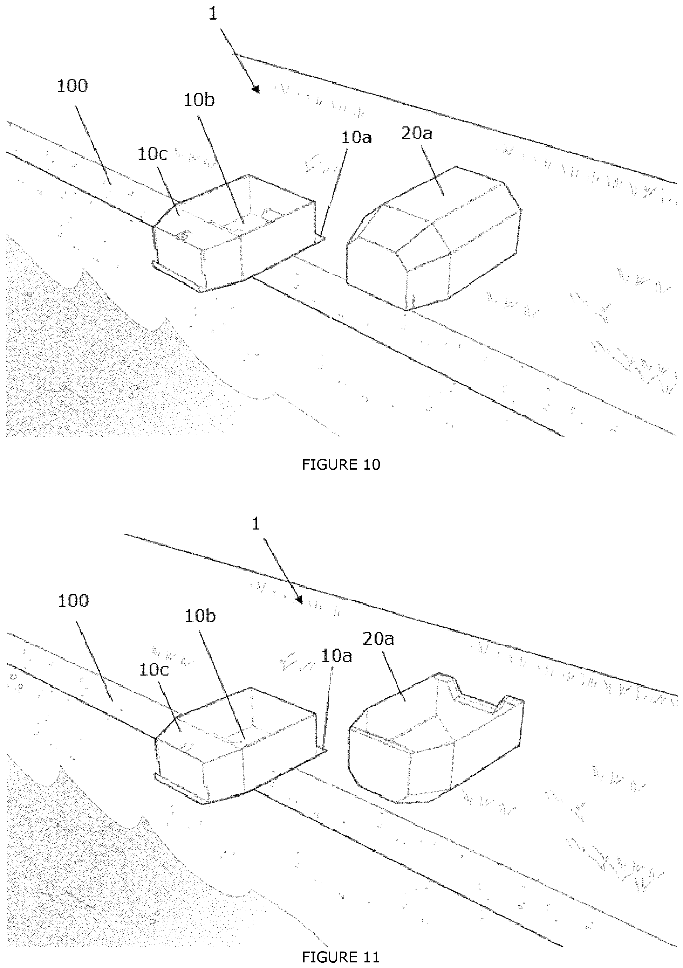

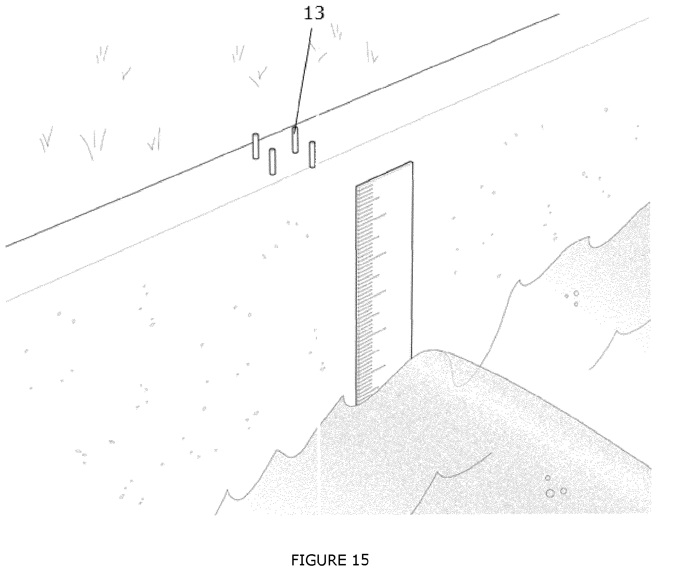

On the other hand, in a first preferred configuration, the anti-vandalism mounting system (1) for monitoring the water physical variables in natural and artificial open channels described by the present invention, is positioned and installed, according to what is shown in Figures 10 to 13, on one edge of the channel (100), wherein a first member (10) is arranged, made up of a base (10a), a first element (10b) and a second element (10c), in a position that allows a part of the base (10a) of said first member (10) to be on the surface of the water. The section of the first member (10) that remains on the edge of the channel (100) is fixed to it by at least three anchoring means (13) which can be seen in detail in Figure 15.

In the same Figures 10 to 13 it is also observed that the system (1) has a second member (20) which is installed in the system (1) in a pivoting manner to the first member (10) or in such a way that can be completely removed over the first member (10).

The assembly of the first and second member configures inside the system (1) a series of compartments that allow housing all the devices, sensors, storage elements and/or energy transformation, among others, necessary for the operation of the system (1), in a secure and inaccessible way for third parties who wish to access it, due to the way in which said members are anchored to the system (1).

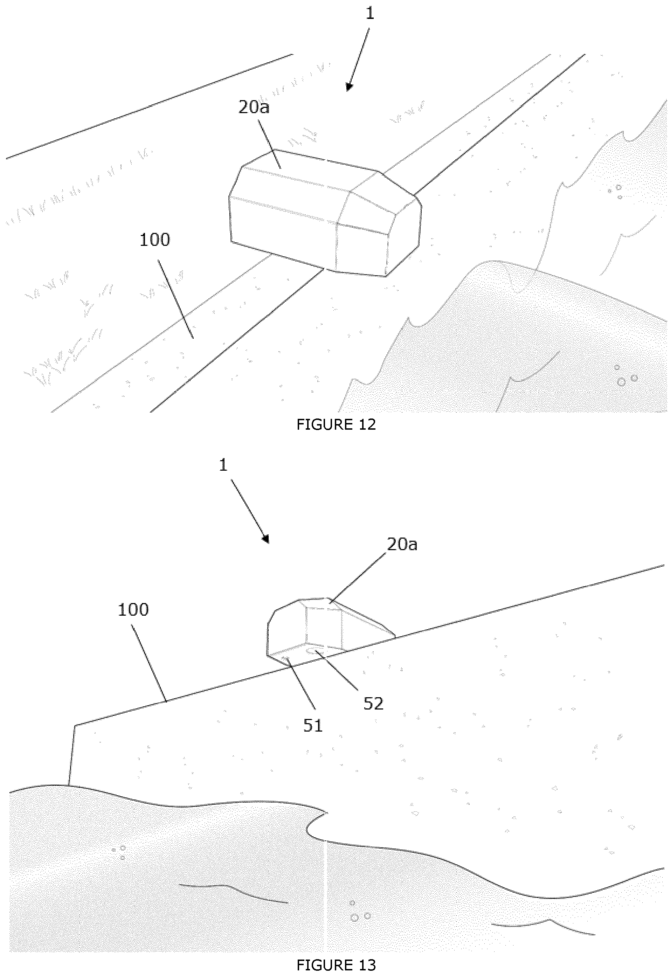

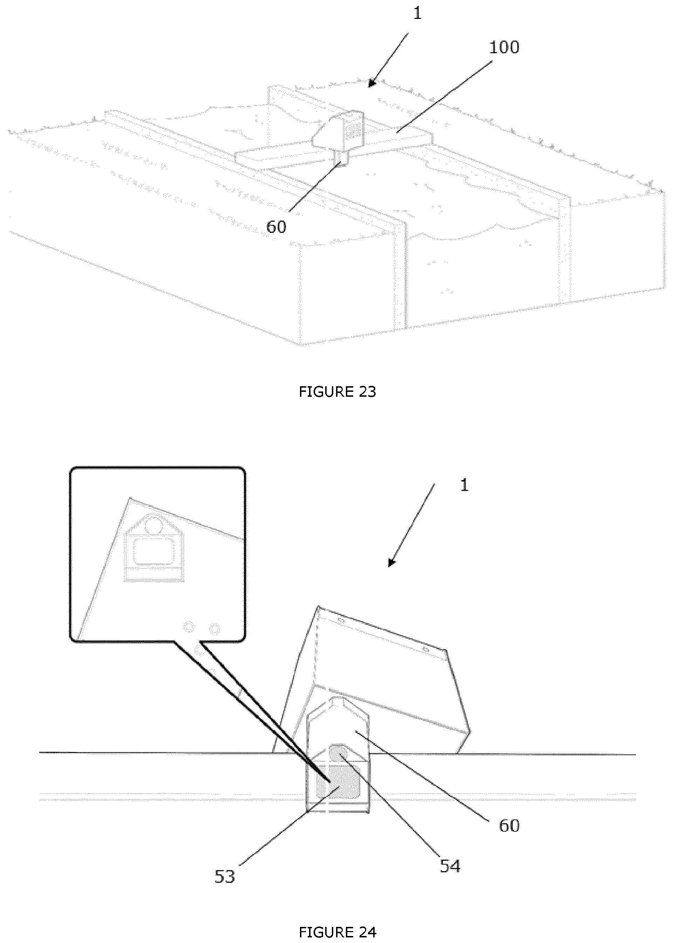

Finally, in Figure 13 it is observed that the system (1) also comprises a housing element (51) and a camera (52), wherein the housing element (51) comprises inside a radar or ultrasonic water height measurement sensor (53) and a device for measuring the surface velocity and runoff height (54) in the channel (see Figure 24). From the arrangement of the ultrasonic sensor (53) within the housing element (51), there is an effect not expected a priori, added to the effect of thermal insulation within the system (1) which allows the thermocouples inside or outside the sensor (53) to be subjected to lower temperature changes and closer to the temperature of the air mass between the sensor (53) and the water surface. In this way, a more accurate measurement can be achieved with less exposure to temperature changes that reflect incorrect reference temperatures when calculating the distance from the ultrasound wave transit time measurement. This is a technical improvement that none of the currently offered solutions describe or suggest for measurements.

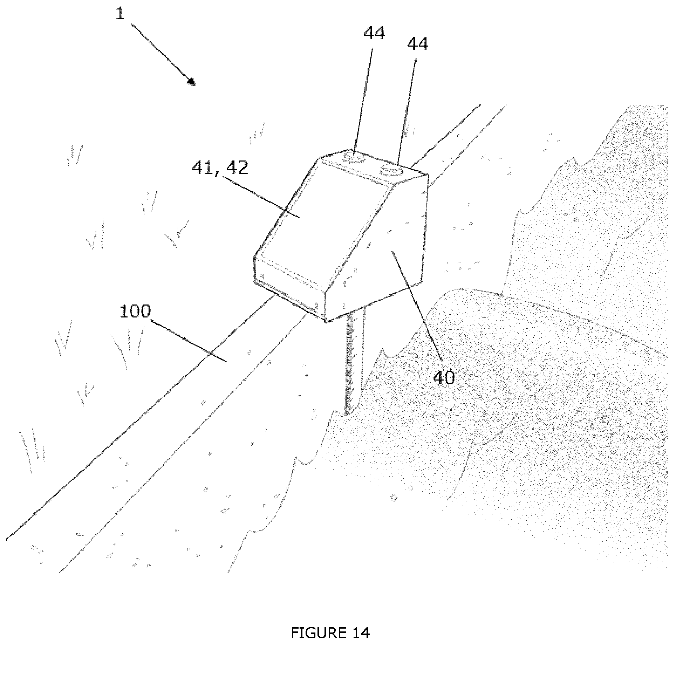

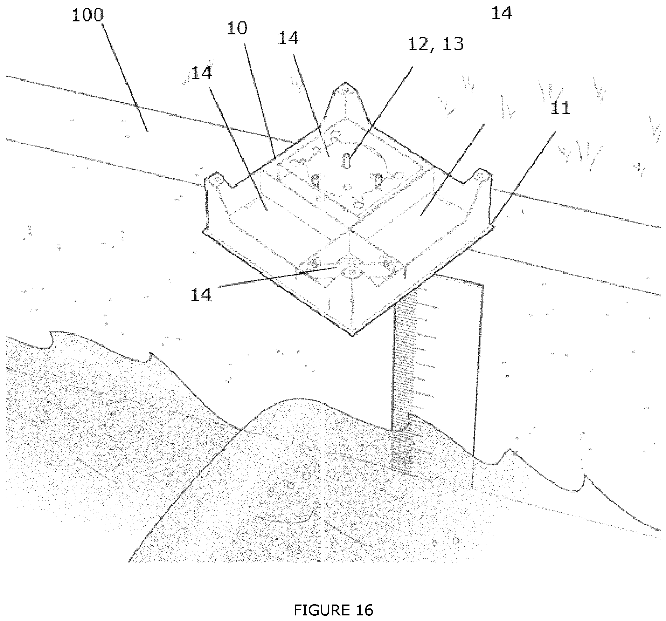

In relation to Figure 14, it shows a second preferred configuration of the technology wherein the anti-vandalism mounting system (1) for monitoring the water physical variables in natural and artificial open channels is also positioned and installed on an edge of the channel (100), wherein, unlike the first preferred configuration, a base (11) of a first member (10) is arranged in a position that allows a part of the base (11) of said first member (10) to be on the surface of the water (see Figure 16). As for the first configuration described, the section of the first member (10) that remains on the edge of the channel (100) is fixed to it by at least three anchoring means (13), which can be seen in detail in Figure 15.

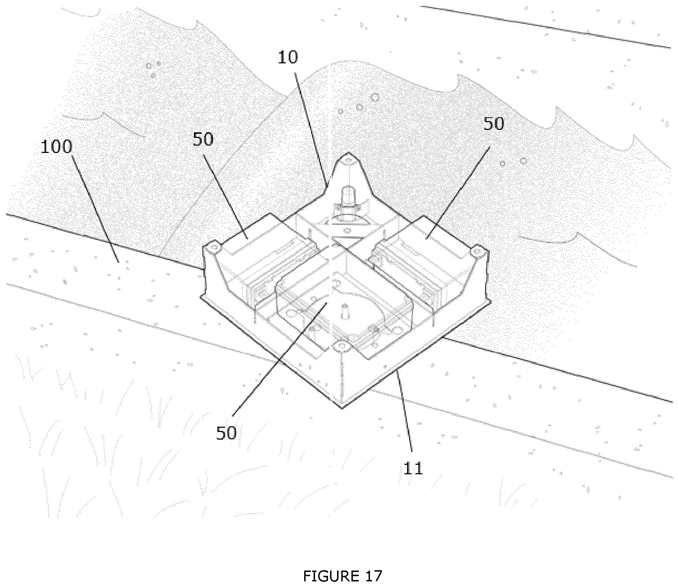

Another important difference between the first configuration and the second preferred configuration described in Figure 14 is related to the fact that the system (1) in this last configuration is made up of four members (10, 20, 30, 40), which are anchored in this same order to form the system (1). The details of the anchors of each of these members can be seen in greater detail in Figures 17, 18, 19 and 20.

In this sense, as can be seen in Figure 14, the fourth member (40) has a different shape in order to be able to receive an energy generating device such as a photovoltaic solar panel.

As in the first preferred configuration, the assembly of the members (10, 20, 30, 40) configures a series of compartments inside the system (1) that allow housing all the devices, sensors, storage elements and/or transformation of energy, among others, necessary for the operation of the system (1), also providing a safe solution and inaccessible for third parties who wish to access it, due to the way in which said members are anchored to the system (1).

The way in which the first member (10) is fixed by the anchoring means (13) can be seen in Figure 16 wherein said anchoring means (13) pass through at least three holes (12) of the first member (10) thereby fixing it to the edge surface of the channel (100).

The number of anchoring means (13) necessary to fix the first member (10) to the installation surface (100) will vary depending on the difficulties present in the ground, such as defects, ironwork or stones in the concrete whereby the number of said anchoring means (13) will generally be between at least three anchoring means (13) and nine anchoring means (13).

In the same Figure 16, as well as in Figure 17, four compartments (14) can be seen, in which the different devices (50) that allow the operation of the system and the measurement of the physical variables of the water are arranged, among which there are a battery, an anti-humidity device, an energy, measurement and telecommunications controller device, an ultrasonic sensor, a camera, and a wireless communication antenna which can operate through 2G, 3G, 4G, etc. cellular networks, and/or through independent wireless networks (5Ghz, 24Ghz band or similar). Together, these devices (50) allow the system to measure remotely, without the need for the presence of the user who can receive the measurements made by the system through a computer, smartphone, or any other means capable of receiving information via the Internet or bluetooth.

Having the possibility of transmitting information wirelessly allows the system of the invention to connect several of these systems (1) along a channel wherein one of them can act as a gateway for the rest of the systems (1). This makes it possible to have a main system (gateway) that contains all the particularities described for the invention and additional smaller systems (1) which only obtain essential information from the channel to be sent to the main system (1), so that it consolidates the information received and sends it to the user. Information can be sent between systems by means of LoRa-type radio waves or any other similar means that allows information to be sent wirelessly.

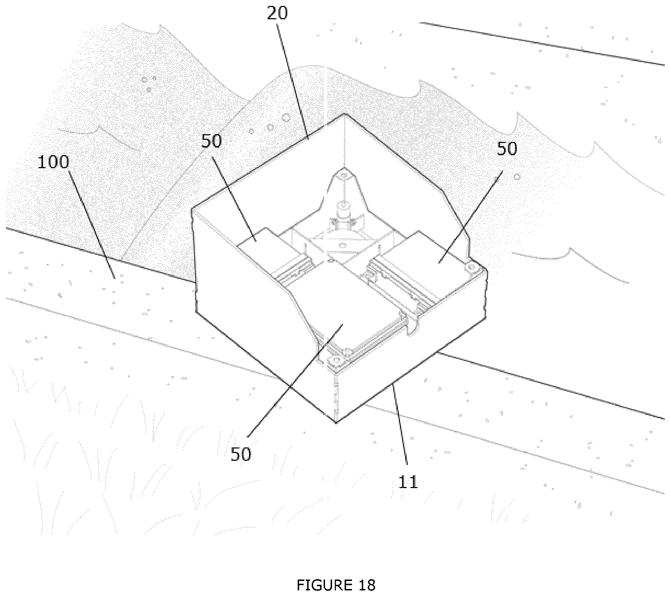

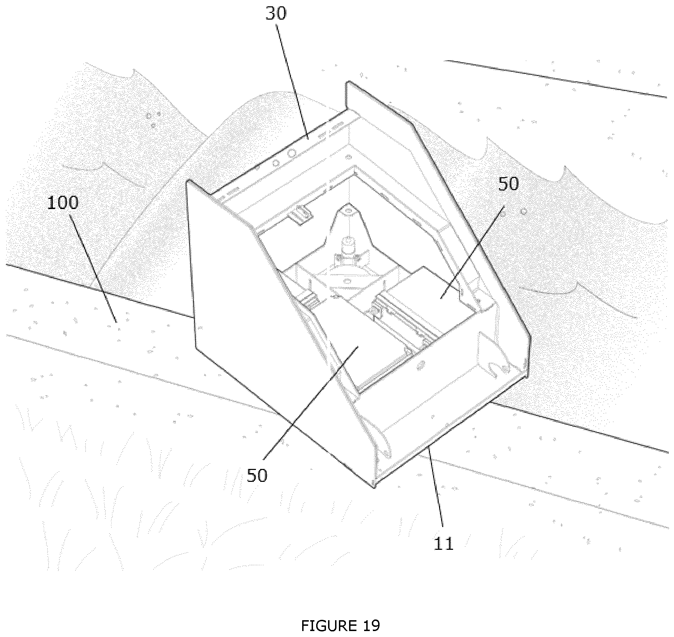

Figures 18 and 19 show the arrangement of the second (20) and third member (30) in the system (1), respectively. The second member (20) is fixed around the lateral faces of the first member (10) through a plurality of anchoring means. Likewise, the third member (30) is attached to the first (10) and second member (20) from their inner faces also by anchoring means, allowing that said anchoring means cannot be detached from the outside.

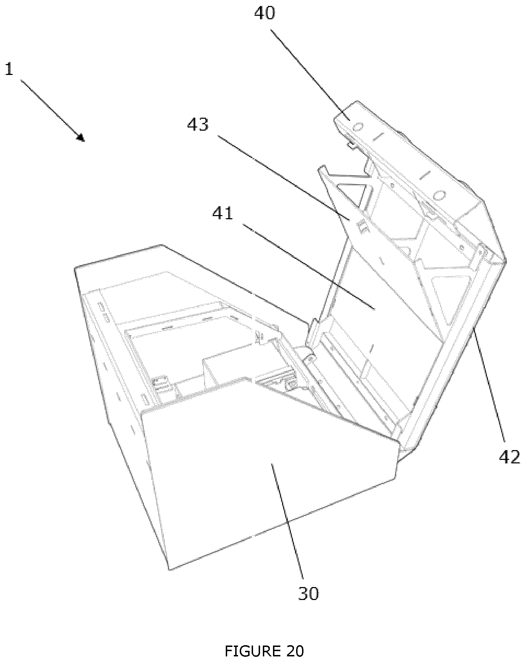

In relation to Figure 20, this shows the arrangement of the fourth member (40) in the system (1) through pivoting means that allow said fourth member (40) to open to access the devices (50) of the system (1). In addition, the provision of the energy generating device (41) is observed, corresponding to a photovoltaic solar panel attached to the inner surface of the fourth member (40) which allows the system (1) to operate as a single individual unit avoiding the placement of other elements outside the system (1), being exposed to vandalism. A rear support (43) is also observed which supports the photovoltaic solar panel (41) together with the impact resistant cover arranged on it, so that they are perfectly positioned within the system (1).

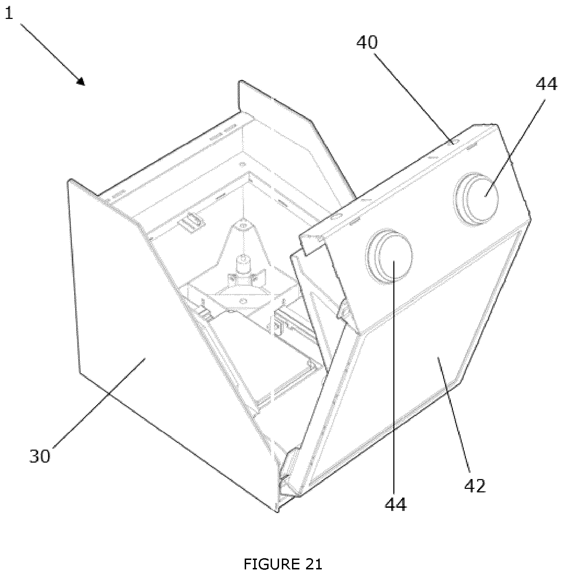

Likewise, Figure 21 shows an isometric view of the system (1) wherein the front part of the fourth member (40) can be seen. An impact resistant cover (42) is placed on said front surface which covers the surface of the photovoltaic solar panel (41), in order to prevent it from being damaged while allowing it to continue receiving solar radiation normally. Additionally, two safety elements (44) are arranged after the impact resistant cover (41) which are hooked to the third member (30) thus leaving both members (30, 40) fixed and secured preventing third parties from accessing the system components (1).

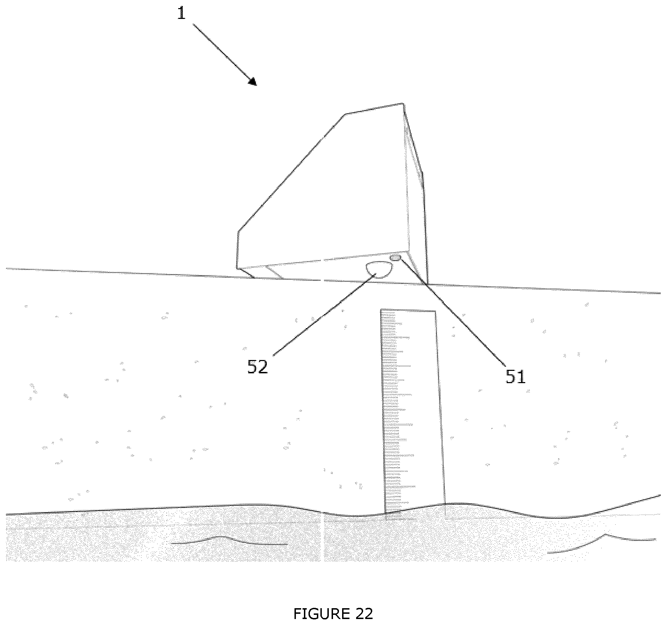

Regarding Figure 22, it shows a view from the surface of the water towards the system (1) wherein the arrangement of a housing element (51) and a camera (52) can be seen which comprise the same advantages described for the first preferred configuration, wherein the housing element (51) comprises inside a radar or ultrasonic water height measurement sensor (53) and a device for measuring the surface velocity and runoff height (54) in the channel. From the arrangement of the ultrasonic sensor (53) inside the housing element (51) there is an effect not expected a priori, added to the effect of thermal insulation within the system (1) which allows the thermocouples inside the sensor (53) to be subjected to lower temperature changes and closer to the temperature of the air mass between the sensor (53) and the water surface. In this way, a more accurate measurement can be achieved with less exposure to temperature changes that reflect incorrect reference temperatures when calculating the distance from the ultrasound wave transit time measurement. This is a technical improvement that none of the currently offered solutions describe or suggest for measurements.

Regarding Figure 23, it shows an isometric view of the system, where a mounting on a bridge (100) that crosses the channel can be seen which acts as an installation surface. From this arrangement, an additional module (60) may be positioned which is mounted in the lower surface of the system (1) containing both the height sensors (53) and the surface speed measurement device (54) of the channel.

Finally, with respect to Figure 24, it shows a detail of the additional module (60) which has anti-vandalism and technical characteristics, and which supports the sensors and devices (53, 53) that allow measuring the height and superficial velocity of the water.

The arrangement shown in Figures 23 and 24 prevents interference from occurring between the radar or ultrasonic water height measurement sensor (53) and the channel wall when it is arranged in the housing element (51) which frequently occurs in several existing solutions in the state of the art resulting in erroneous information to the system operator leading him to make bad decisions that may finally lead to implement expensive solutions due to bad measurements taken.

### NUMERICAL REFERENCES

- 1: An anti-vandalism mounting system for monitoring water physical variables in open channels
- 10: First member
- 10a: Base
- 10b: First element
- 10c: Second element
- 11: Base
- 12: Perforations
- 13: Anchoring means
- 14: Compartments
- 20: Second member
- 30: Third member
- 40: Fourth member
- 41: Energy generating device
- 42: Impact resistant cover
- 43: Rear support
- 44: Safety devices
- 50: Devices
- 51: Housing element
- 52: Camera
- 53: Radar or ultrasonic water height measurement sensor
- 54: Device for measuring surface velocity and runoff height
- 60: Additional module
- 100: Installation surface

## Claims

1. An anti-vandalism mounting system for monitoring the physical variables of water in open channels, wherein it comprises:
- a first member (10), comprising a base with a plurality of perforations for introducing a plurality of anchoring means (13) for fixing the first member to an installation surface (100) of the system;
- a second member (20), which is fixed on the first member of the system by means of a plurality of anchoring means;
- a third member (30), arranged externally which is attached to the first and second members from inside the system by anchoring means; and
- a fourth member (40), pivotally arranged at the bottom of the third member;
wherein the first member comprises a plurality of compartments (14) to house a plurality of devices (50) for the operation of the system and for the monitoring of physical variables to be protected by the system; and
wherein the system comprises an energy generating device (41) and a plurality of safety devices (44), so that the fourth member is fixed to the third member of the system.

2. The system according to claim 1, wherein the devices for the operation of the system and for the monitoring of physical variables correspond to a plurality of batteries, at least one anti-humidity device, at least one energy, measurement and telecommunications controller device, at least one ultrasonic sensor, and at least one wireless communication antenna.

3. The system according to claim 1, wherein the power generating device is a photovoltaic solar panel, wherein the power generating device is comprised by the fourth member which also comprises an impact resistant cover on said power generator, wherein both the power generating device and the impact resistant cover are supported by a rear support of the power generating device.

4. The system according to claim 2, wherein the devices for the operation of the system and for the monitoring of physical variables also comprise at least one camera (52) to monitor the status of the channel, to verify the presence of garbage or foreign objects, also operating as a means for verifying the height of the water.

5. The system according to claim 1, wherein it further comprises at least one element for measuring the height of water, mounted on the third member, wherein the element for measuring the height of water is a radar device (53).

6. The system according to claim 1, wherein it further comprises a module (54) for measuring the runoff velocity profile in the channel laterally or at the bottom of the channel comprising two parts fixed to the inner members of the system and that supports a device with a plurality of transducers for the measurement of average speed by Doppler effect, transit time or other similar method, the module extending to the wall of the channel or the bottom, which is fixed to said channel wall by a plurality of anchoring means.

7. The system according to claim 1, wherein it also comprises a housing element (51) above the channel fixed to the inner members of the system by anchoring means, wherein a radar or ultrasonic water height measurement sensor (53) and a device for measuring the surface velocity and runoff height (54) in the channel are mounted at its end.

8. The system according to claim 1, wherein it also comprises an additional module (60) mounted on the lower part of the system fixed to it through anchoring means, wherein a radar or ultrasonic water height measurement sensor and a device for measuring the surface speed and runoff height in the channel are arranged inside it.

9. The system according to claim 1, wherein it further comprises an arm, preferably horizontal, mounted on the lower part of the system, fixed at one of its ends to said lower part through anchoring means, wherein at its other end an additional module is mounted, fixed to it through anchoring means, wherein a radar or ultrasonic water height measurement sensor and a device for measuring surface speed and runoff height in the channel are arranged inside it.

10. The system according to claim 1, wherein the installation surface of the system corresponds to:
the upper edge of one of the channel walls; or
a bridge which crosses the channel transversely and which is mounted on the upper edges of the channel walls through anchoring means.

11. A procedure for assembling an anti-vandalism mounting system for monitoring the physical variables of water in open channels, wherein it comprises the following stages:
(i) fixing a first member (10) to an installation surface (100) of the system by inserting a plurality of anchoring means (13) into a plurality of perforations in said first member;
(ii) fixing a second member (20) to the first member by means of a plurality of anchoring means;
(iii) fixing a third member (30), arranged externally, to the first and second members, from inside the system by anchoring means;
(iv) pivotally arranging a fourth member (40) in the lower part of the third member;
(v) housing a plurality of devices (50) to be protected by the system in a plurality of compartments of the first member;
(vi) providing an energy generating device (41) in the system; and
(vii) providing a plurality of safety devices (44) in the fourth member, so that it is fixed to the third member of the system.

12. The procedure according to claim 11, wherein the stage of providing an energy generating device in the system further comprises arranging said energy generating device together with an impact resistant cover on said energy generating device in the fourth member, wherein the energy generating device corresponds to a photovoltaic solar panel;
wherein the procedure further comprises providing a rear support for the photovoltaic solar panel in the fourth member to support said photovoltaic solar panel and the impact resistant cover.

13. The procedure according to claim 11, wherein it further comprises:
mounting at least one element for measuring the height of water in the third member; and
mounting a module for measuring the runoff velocity profile in the channel, laterally or at the bottom of the channel, on the inner members of the system, comprising two parts fixed to said inner members and supporting a device with a plurality of transducers for the measurement of average speed by Doppler effect, transit time or other similar method, the module extending to the wall or the bottom of the channel which is fixed to said wall of the channel by a plurality of anchoring means.

14. The procedure according to claim 11, wherein it further comprises:
mounting a housing element over the channel on the inner members of the system, fixed to said inner members by anchoring means, wherein a radar or ultrasonic water height measurement sensor and a device for measuring the surface velocity and runoff height in the channel are mounted at its end; or
mounting an additional module to the lower part of the system, fixed to it through anchoring means, wherein a radar or ultrasonic water height measurement sensor and a device for measuring the surface velocity and runoff height in the channel are arranged inside it.

15. The procedure according to claim 11, wherein it further comprises mounting an arm, preferably horizontal, to the lower part of the system, fixed at one of its ends to said lower part through anchoring means, wherein an additional module is mounted at its other end, fixed to it through anchoring means, wherein a radar or ultrasonic water height measurement sensor and a device for measuring surface speed and runoff height in the channel are arranged inside it.

## Patentansprüche

1. Ein Anti-Vandalismus-Montagesystem zur Überwachung der physikalischen Variablen von Wasser in offenen Kanälen, wobei es umfasst:
- ein erstes Element (10), das eine Basis mit einer Vielzahl von Perforationen zum Einführen einer Vielzahl von Verankerungsmitteln (13) zum Fixieren des ersten Elements an einer Installationsoberfläche (100) des Systems umfasst;
- ein zweites Element (20), das am ersten Element des Systems mittels einer Vielzahl von Verankerungsmitteln befestigt ist;
- ein drittes Element (30), das extern angeordnet ist und von innen am ersten und zweiten Element des Systems durch Verankerungsmittel befestigt ist; und
- ein viertes Element (40), das schwenkbar am unteren Ende des dritten Elements angeordnet ist;
wobei das erste Element eine Vielzahl von Fächern (14) zur Unterbringung einer Vielzahl von Geräten (50) für den Betrieb des Systems und zur Überwachung der physikalischen Variablen, die durch das System geschützt werden sollen, umfasst; und
wobei das System ein Energieerzeugungsgerät (41) und eine Vielzahl von Sicherheitsgeräten (44) umfasst, sodass das vierte Element am dritten Element des Systems befestigt ist.

2. Das System gemäß Anspruch 1, wobei die Geräte für den Betrieb des Systems und zur Überwachung der physikalischen Variablen einer Vielzahl von Batterien, mindestens einem Antihumiditätsgerät, mindestens einem Energie-, Mess- und Telekommunikationssteuergerät, mindestens einem Ultraschallsensor und mindestens einer drahtlosen Kommunikationsantenne entsprechen.

3. Das System gemäß Anspruch 1, wobei das Energieerzeugungsgerät ein photovoltaisches Solarpanel ist, wobei das Energieerzeugungsgerät im vierten Element enthalten ist, das auch eine schlagfeste Abdeckung über dem genannten Energieerzeuger umfasst, wobei sowohl das Energieerzeugungsgerät als auch die schlagfeste Abdeckung von einer Rückstütze des Energieerzeugungsgeräts getragen werden.

4. Das System gemäß Anspruch 2, wobei die Geräte für den Betrieb des Systems und zur Überwachung der physikalischen Variablen auch mindestens eine Kamera (52) umfassen, um den Zustand des Kanals zu überwachen, um das Vorhandensein von Müll oder Fremdkörpern zu überprüfen, und auch als Mittel zur Überprüfung der Wasserstand zu fungieren.

5. Das System gemäß Anspruch 1, wobei es zusätzlich mindestens ein Element zur Messung der Wasserstand umfasst, das am dritten Element montiert ist, wobei das Element zur Messung der Wasserstand ein Radargerät (53) ist.

6. Das System gemäß Anspruch 1, wobei es zusätzlich ein Modul (54) zur Messung des Abflussgeschwindigkeitsprofils im Kanal seitlich oder am Boden des Kanals umfasst, das aus zwei Teilen besteht, die an den inneren Elementen des Systems befestigt sind und ein Gerät mit einer Vielzahl von Transducern zur Messung der Durchschnittsgeschwindigkeit durch Dopplereffekt, Transitzeit oder andere ähnliche Methoden unterstützen, wobei das Modul bis zur Wand des Kanals oder zum Boden reicht, das mit einer Vielzahl von Verankerungsmitteln an der genannten Kanalwand befestigt ist.

7. Das System gemäß Anspruch 1, wobei es auch ein Gehäuselement (51) über dem Kanal umfasst, das an den inneren Elementen des Systems durch Verankerungsmittel befestigt ist, wobei ein Radar- oder Ultraschall-Wassertiefenmesssensor (53) und ein Gerät zur Messung der Oberflächengeschwindigkeit und der Abflusshöhe (54) im Kanal an seinem Ende montiert sind.

8. Das System gemäß Anspruch 1, wobei es auch ein zusätzliches Modul (60) umfasst, das an der unteren Seite des Systems befestigt ist, wobei ein Radar- oder Ultraschall-Wassertiefenmesssensor und ein Gerät zur Messung der Oberflächengeschwindigkeit und der Abflusshöhe im Kanal darin angeordnet sind.

9. Das System gemäß Anspruch 1, wobei es zusätzlich einen Arm, vorzugsweise horizontal, umfasst, der an der unteren Seite des Systems montiert ist, wobei er an einem seiner Enden durch Verankerungsmittel an der genannten unteren Seite befestigt ist, wobei an seinem anderen Ende ein zusätzliches Modul montiert ist, das durch Verankerungsmittel daran befestigt ist, wobei ein Radar- oder Ultraschall-Wassertiefenmesssensor und ein Gerät zur Messung der Oberflächengeschwindigkeit und der Abflusshöhe im Kanal darin angeordnet sind.

10. Das System gemäß Anspruch 1, wobei die Installationsoberfläche des Systems entspricht:
der oberen Kante einer der Kanalwände; oder
einer Brücke, die den Kanal quer überquert und die an den oberen Kanten der Kanalwände durch Verankerungsmittel montiert ist.

11. Ein Verfahren zum Zusammenbauen eines Anti-Vandalismus-Montagesystems zur Überwachung der physikalischen Variablen von Wasser in offenen Kanälen, wobei es die folgenden Phasen umfasst:
(i) ein erstes Element (10) an einer Installationsoberfläche (100) des Systems zu befestigen, indem eine Vielzahl von Verankerungsmitteln (13) in eine Vielzahl von Perforationen in dem genannten ersten Element eingeführt wird;
(ii) ein zweites Element (20) am ersten Element mittels einer Vielzahl von Verankerungsmitteln zu befestigen;
(iii) ein drittes Element (30), das extern angeordnet ist, von innen am ersten und zweiten Element des Systems durch Verankerungsmittel zu befestigen;
(iv) ein viertes Element (40) im unteren Teil des dritten Elements schwenkbar anzuordnen;
(v) eine Vielzahl von Geräten (50), die durch das System geschützt werden sollen, in einer Vielzahl von Fächern des ersten Elements unterzubringen;
(vi) ein Energieerzeugungsgerät (41) im System bereitzustellen; und
(vii) eine Vielzahl von Sicherheitsgeräten (44) im vierten Element bereitzustellen, sodass es am dritten Element des Systems befestigt ist.

12. Das Verfahren gemäß Anspruch 11, wobei die Phase der Bereitstellung eines Energieerzeugungsgeräts im System zusätzlich umfasst, das genannte Energieerzeugungsgerät zusammen mit einer schlagfesten Abdeckung über dem genannten Energieerzeugungsgerät im vierten Element anzuordnen, wobei das Energieerzeugungsgerät einem photovoltaischen Solarpanel entspricht;
wobei das Verfahren zusätzlich umfasst, eine Rückstütze für das photovoltaische Solarpanel im vierten Element bereitzustellen, um das genannte photovoltaische Solarpanel und die schlagfeste Abdeckung zu stützen.

13. Das Verfahren gemäß Anspruch 11, wobei es zusätzlich umfasst:
mindestens ein Element zur Messung der Wasserstand im dritten Element zu montieren; und
ein Modul zur Messung des Abflussgeschwindigkeitsprofils im Kanal, seitlich oder am Boden des Kanals, an den inneren Elementen des Systems zu montieren, das aus zwei Teilen besteht, die an den genannten inneren Elementen befestigt sind und ein Gerät mit einer Vielzahl von Transducern zur Messung der Durchschnittsgeschwindigkeit durch Dopplereffekt, Transitzeit oder andere ähnliche Methoden unterstützen, wobei das Modul bis zur Wand oder zum Boden des Kanals reicht, das mit einer Vielzahl von Verankerungsmitteln an der genannten Wand des Kanals befestigt ist.

14. Das Verfahren gemäß Anspruch 11, wobei es zusätzlich umfasst:
ein Gehäuselement über dem Kanal an den inneren Elementen des Systems zu montieren, das an den genannten inneren Elementen durch Verankerungsmittel befestigt ist, wobei ein Radar- oder Ultraschall-Wassertiefenmesssensor und ein Gerät zur Messung der Oberflächengeschwindigkeit und der Abflusshöhe im Kanal an seinem Ende montiert sind; oder
ein zusätzliches Modul an der unteren Seite des Systems zu montieren, das durch Verankerungsmittel daran befestigt ist, wobei ein Radar- oder Ultraschall-Wassertiefenmesssensor und ein Gerät zur Messung der Oberflächengeschwindigkeit und der Abflusshöhe im Kanal darin angeordnet sind.

15. Das Verfahren nach Anspruch 11, wobei es ferner umfasst, einen Arm, vorzugsweise horizontal, an der unteren Seite des Systems zu montieren, der an einem Ende mit der genannten unteren Seite durch Verankerungsmittel fixiert ist, wobei an seinem anderen Ende ein zusätzliches Modul montiert ist, das durch Verankerungsmittel mit ihm verbunden ist, wobei ein Radar- oder Ultraschall-Wassertiefenmesssensor und ein Gerät zur Messung der Oberflächengeschwindigkeit und der Abflusshöhe im Kanal darin angeordnet sind.

## Revendications

1. Un système de montage anti-vandalisme pour surveiller les variables physiques de l'eau dans des canaux ouverts, comprenant :
- un premier membre (10), comprenant une base avec une pluralité de perforations pour introduire une pluralité de moyens d'ancrage (13) pour fixer le premier membre à une surface d'installation (100) du système ;
- un deuxième membre (20), qui est fixé au premier membre du système par le biais d'une pluralité de moyens d'ancrage ;
- un troisième membre (30), disposé à l'extérieur, qui est attaché aux premier et deuxième membres de l'intérieur du système par des moyens d'ancrage ; et
- un quatrième membre (40), disposé de manière pivotante au bas du troisième membre ;
où le premier membre comprend une pluralité de compartiments (14) pour abriter une pluralité de dispositifs (50) pour le fonctionnement du système et pour la surveillance des variables physiques à protéger par le système ; et
où le système comprend un dispositif de génération d'énergie (41) et une pluralité de dispositifs de sécurité (44), de sorte que le quatrième membre soit fixé au troisième membre du système.

2. Le système selon la revendication 1, où les dispositifs pour le fonctionnement du système et pour la surveillance des variables physiques correspondent à une pluralité de batteries, au moins un dispositif anti-humidité, au moins un dispositif de contrôle d'énergie, de mesure et de télécommunications, au moins un capteur ultrasonique, et au moins une antenne de communication sans fil.

3. Le système selon la revendication 1, où le dispositif de génération d'énergie est un panneau solaire photovoltaïque, où le dispositif de génération d'énergie est compris dans le quatrième membre qui comprend également un couvercle résistant aux impacts sur ledit générateur d'énergie, où à la fois le dispositif de génération d'énergie et le couvercle résistant aux impacts sont soutenus par un support arrière du dispositif de génération d'énergie.

4. Le système selon la revendication 2, où les dispositifs pour le fonctionnement du système et pour la surveillance des variables physiques comprennent également au moins une caméra (52) pour surveiller l'état du canal, pour vérifier la présence de déchets ou d'objets étrangers, fonctionnant également comme un moyen de vérifier la hauteur de l'eau.

5. Le système selon la revendication 1, où il comprend également au moins un élément pour mesurer la hauteur de l'eau, monté sur le troisième membre, où l'élément pour mesurer la hauteur de l'eau est un dispositif radar (53).

6. Le système selon la revendication 1, où il comprend également un module (54) pour mesurer le profil de vitesse d'écoulement dans le canal latéralement ou au fond du canal comprenant deux parties fixées aux membres intérieurs du système et soutenant un dispositif avec une pluralité de transducteurs pour la mesure de la vitesse moyenne par effet Doppler, temps de transit ou autre méthode similaire, le module s'étendant jusqu'à la paroi du canal ou le fond, qui est fixé à ladite paroi du canal par une pluralité de moyens d'ancrage.

7. Le système selon la revendication 1, où il comprend également un élément de logement (51) au-dessus du canal fixé aux membres intérieurs du système par des moyens d'ancrage, où un capteur de mesure de hauteur d'eau radar ou ultrasonique (53) et un dispositif pour mesurer la vitesse de surface et la hauteur d'écoulement (54) dans le canal sont montés à son extrémité.

8. Le système selon la revendication 1, où il comprend également un module supplémentaire (60) monté sur la partie inférieure du système fixé à celui-ci par des moyens d'ancrage, où un capteur de mesure de hauteur d'eau radar ou ultrasonique et un dispositif pour mesurer la vitesse de surface et la hauteur d'écoulement dans le canal sont disposés à l'intérieur.

9. Le système selon la revendication 1, où il comprend également un bras, de préférence horizontal, monté sur la partie inférieure du système, fixé à l'une de ses extrémités à ladite partie inférieure par des moyens d'ancrage, où à son autre extrémité un module supplémentaire est monté, fixé à celui-ci par des moyens d'ancrage, où un capteur de mesure de hauteur d'eau radar ou ultrasonique et un dispositif pour mesurer la vitesse de surface et la hauteur d'écoulement dans le canal sont disposés à l'intérieur.

10. Le système selon la revendication 1, où la surface d'installation du système correspond à :
le bord supérieur de l'un des murs du canal ; ou
un pont qui traverse le canal transversalement et qui est monté sur les bords supérieurs des murs du canal par des moyens d'ancrage.

11. Une procédure pour assembler un système de montage anti-vandalisme pour surveiller les variables physiques de l'eau dans des canaux ouverts, comprenant les étapes suivantes :
(i) fixer un premier membre (10) à une surface d'installation (100) du système en insérant une pluralité de moyens d'ancrage (13) dans une pluralité de perforations dans ledit premier membre ;
(ii) fixer un deuxième membre (20) au premier membre par le biais d'une pluralité de moyens d'ancrage ;
(iii) fixer un troisième membre (30), disposé à l'extérieur, aux premier et deuxième membres, de l'intérieur du système par des moyens d'ancrage ;
(iv) disposer de manière pivotante un quatrième membre (40) dans la partie inférieure du troisième membre ;
(v) abriter une pluralité de dispositifs (50) à protéger par le système dans une pluralité de compartiments du premier membre ;
(vi) fournir un dispositif de génération d'énergie (41) dans le système ; et
(vii) fournir une pluralité de dispositifs de sécurité (44) dans le quatrième membre, de sorte qu'il soit fixé au troisième membre du système.

12. La procédure selon la revendication 11, où l'étape de fournir un dispositif de génération d'énergie dans le système comprend également disposer ledit dispositif de génération d'énergie avec un couvercle résistant aux impacts sur ledit dispositif de génération d'énergie dans le quatrième membre, où le dispositif de génération d'énergie correspond à un panneau solaire photovoltaïque ;
où la procédure comprend également fournir un support arrière pour le panneau solaire photovoltaïque dans le quatrième membre pour soutenir ledit panneau solaire photovoltaïque et le couvercle résistant aux impacts.

13. La procédure selon la revendication 11, où elle comprend également :
monter au moins un élément pour mesurer la hauteur de l'eau dans le troisième membre ; et
monter un module pour mesurer le profil de vitesse d'écoulement dans le canal, latéralement ou au fond du canal, sur les membres intérieurs du système, comprenant deux parties fixées à ces membres intérieurs et soutenant un dispositif avec une pluralité de transducteurs pour la mesure de la vitesse moyenne par effet Doppler, temps de transit ou autre méthode similaire, le module s'étendant jusqu'à la paroi ou le fond du canal qui est fixé à ladite paroi du canal par une pluralité de moyens d'ancrage.

14. La procédure selon la revendication 11, où elle comprend également :
monter un élément de logement au-dessus du canal sur les membres intérieurs du système, fixé à ces membres intérieurs par des moyens d'ancrage, où un capteur de mesure de hauteur d'eau radar ou ultrasonique et un dispositif pour mesurer la vitesse de surface et la hauteur d'écoulement dans le canal sont montés à son extrémité ; ou
monter un module supplémentaire à la partie inférieure du système, fixé à celui-ci par des moyens d'ancrage, où un capteur de mesure de hauteur d'eau radar ou ultrasonique et un dispositif pour mesurer la vitesse de surface et la hauteur d'écoulement dans le canal sont disposés à l'intérieur.

15. La procédure selon la revendication 11, dans laquelle elle comprend en outre le montage d'un bras, de préférence horizontal, à la partie inférieure du système, fixé à l'une de ses extrémités à ladite partie inférieure par des moyens d'ancrage, dans laquelle un module supplémentaire est monté à son autre extrémité, fixé à celui-ci par des moyens d'ancrage, dans laquelle un capteur de mesure de hauteur d'eau radar ou ultrasonique et un dispositif de mesure de la vitesse de surface et de la hauteur d'écoulement dans le canal sont disposés à l'intérieur.
